# EUROPÄISCHE PATENTSCHRIFT

(11) **EP 1 682 147 B1**
(45) Veröffentlichungstag und Bekanntmachung des Hinweises auf die Patenterteilung: **07.10.2009**
(21) Anmeldenummer: 04790780.3
(22) Anmeldetag: 23.10.2004
(51) Int. Cl.: A61K 31/517, A61P 29/00, A61P 1/00

(54) **VERWENDUNG VON TYROSINKINASE-INHIBITOREN ZUR BEHANDLUNG INFLAMMATORISCHER PROZESSE**
USE OF TYROSINE KINASE INHIBITORS FOR THE TREATMENT OF INFLAMMATORY PROCESSES
UTILISATION D'INHIBITEURS DE TYROSINE-KINASE POUR TRAITER DES PROCESSUS INFLAMMATOIRES

(30) Priorität: 30.10.2003 DE 10350717
(43) Veröffentlichungstag der Anmeldung: 26.07.2006
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: JUNG, Birgit, 88471 Laupheim (DE); PUESCHNER, Hubert, 88400 Biberach (DE)
(74) Vertreter: Hammann, Heinz
(86) Internationale Anmeldenummer: PCT/EP2004/011989
(87) Internationale Veröffentlichungsnummer: WO 2005/041973

(56) Entgegenhaltungen:
- EP-A- 1 184 376
- WO-A-03/082290
- DE-A1- 10 042 058
- DE-A1- 10 042 059
- KITZMANN A.S. ET AL: 'Histologic studies of the intraocular toxicity of imatinib mesylate in rabbits"' EYE Bd. 22, Nr. 5, 2008, Seiten 712 - 714

## Beschreibung

Gegenstand der vorliegenden Erfindung ist die Verwendung von Chinazolinen ausgewählt aus der Gruppe bestehend aus
(1) 4-[(3-Chlor-4-fluor-phenyl)amino]-6-[1-(tert.-butyloxycarbonyl)-piperidin-4-yloxy]-7-methoxy-chinazolin,
(2) 4-[(3-Chlor-4-fluor-phenyl)amino]-6-(trans-4-amino-cyclohexan-1-yloxy)-7-methoxy-chinazolin,
(3) 4-[(3-Chlor-4-fluor-phenyl)amino]-6-(trans-4-methansulfonylamino-cyclohexan-1-yloxy)-7-methoxy-chinazolin,
(4) 4-[(3-Chlor-4-fluor-phenyl)amino]-6-(tetrahydropyran-3-yloxy)-7-methoxy-chinazolin,
(5) 4-[(3-Chlor-4-fluor-phenyl)amino]-6-(1-methyl-piperidin-4-yloxy)-7-methoxy-chinazolin,
(6) 4-[(3-Chlor-4-fluor-phenyl)amino]-6-{1-[(morpholin-4-yl)carbonyl]-piperidin-4-yl-oxy}-7-methoxy-chinazolin,
(7) 4-[(3-Chlor-4-fluor-phenyl)amino]-6-{1-[(methoxymethyl)carbonyl]-piperidin-4-yl-oxy}-7-methoxy-chinazolin,
(8) 4-[(3-Chlor-4-fluor-phenyl)amino]-6-(piperidin-3-yloxy)-7-methoxy-chinazolin,
(9) 4-[(3-Chlor-4-fluor-phenyl)amino]-6-[1-(2-acetylamino-ethyl)-piperidin-4-yloxy]-7-methoxy-chinazoli n,
(10) 4-[(3-Chlor-4-fluor-phenyl)amino]-6-(tetrahydropyran-4-yloxy)-7-ethoxy-chinazolin,
(11) 4-[(3-Chlor-4-fluor-phenyl)amino]-6-((*S*)-tetrahydrofuran-3-yloxy)-7-hydroxy-chinazolin,
(12) 4-[(3-Chlor-4-fluor-phenyl)amino]-6-(tetrahydropyran-4-yloxy)-7-(2-methoxy-ethoxy)-chinazolin,
(13) 4-[(3-Chlor-4-fluor-phenyl)amino]-6-{trans-4-[(dimethylamino)sulfonylamino]-cyclohexan-1-yloxy}-7-methoxy-chinazolin,
(14) 4-[(3-Chlor-4-fluor-phenyl)amino]-6-{trans-4-[(morpholin-4-yl)carbonylamino]-cyclohexan-1-yloxy}-7-methoxy-chinazolin,
(15) 4-[(3-Chlor-4-fluor-phenyl)amino]-6-{trans-4-[(morpholin-4-yl)sulfonylamino]-cyclohexan-1-yloxy}-7-methoxy-chinazolin,
(16) 4-[(3-Chlor-4-fluor-phenyl)amino]-6-(tetrahydropyran-4-yloxy)-7-(2-acetylamino-ethoxy)-chinazolin,
(17) 4-[(3-Chlor-4-fluor-phenyl)amino]-6-(tetrahydropyran-4-yloxy)-7-(2-methansulfonylamino-ethoxy)-chinazolin,
(18) 4-[(3-Chlor-4-fluor-phenyl)amino]-6-{1-[(piperidin-1-yl)carbonyl]-piperidin-4-yloxy}-7-methoxy-chinazolin,
(19) 4-[(3-Chlor-4-fluor-phenyl)amino]-6-(1-aminocarbonylmethyl-piperidin-4-yloxy)-7-methoxy-chinazolin,
(20) 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin,
(21) 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin,
(22) 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin,
(23) 4-[(3-Chlor-4-fluor-phenyl)amino]-6-(trans-4-ethansulfonylamino-cyclohexan-1-yloxy)-7-methoxy-chinazolin,
(24) 4-[(3-Chlor-4-fluor-phenyl)amino]-6-(1-methansulfonyl-piperidin-4-yloxy)-7-ethoxy-chinazolin,
(25) 4-[(3-Chlor-4-fluor-phenyl)amino]-6-(1-methansulfonyl-piperidin-4-yloxy)-7-(2-methoxy-ethoxy)-chinazolin,
(26) 4-[(3-Chlor-4-fluor-phenyl)amino]-6-[1-(2-methoxy-acetyl)-piperidin-4-yloxy]-7-(2-methoxy-ethoxy)-chinazolin,
(27) 4-[(3-Chlor-4-fluor-phenyl)amino]-6-(cis-4-acetylamino-cyclohexan-1-yloxy)-7-methoxy-chinazolin,
(28) 4-[(3-Ethinyl-phenyl)amino]-6-[1-(tert.-butyloxycarbonyl)-piperidin-4-yloxy]-7-methoxy-chinazolin,
(29) 4-[(3-Ethinyl-phenyl)amino]-6-(tetrahydropyran-4-yloxy]-7-methoxy-chinazolin,
(30) 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin,
(31) 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin,
(32) 4-[(3-Chlor-4-fluor-phenyl)amino]-6-{cis-4-[(morpholin-4-yl)carbonylamino]-cyclohexan-1-yloxy}-7-methoxy-chinazolin,
(33) 4-[(3-Chlor-4-fluor-phenyl)amino]-6-{1-[2-(2-oxopyrrolidin-1-yl)ethyl]-piperidin-4-yloxy}-7-methoxy-chinazolin,
(34) 4-[(3-Chlor-4-fluor-phenyl)amino]-6-{1-[(morpholin-4-yl)carbonyl]-piperidin-4-yloxy}-7-(2-methoxy-ethoxy)-chinazolin,
(35) 4-[(3-Ethinyl-phenyl)amino]-6-(1-acetyl-piperidin-4-yloxy)-7-methoxy-chinazolin,
(36) 4-[(3-Ethinyl-phenyl)amino]-6-(1-methyl-piperidin-4-yloxy)-7-methoxy-chinazolin,
(37) 4-[(3-Ethinyl-phenyl)amino]-6-(1-methansulfonyl-piperidin-4-yloxy)-7-methoxy-chinazolin,
(38) 4-[(3-Chlor-4-fluor-phenyl)amino]-6-(1-methyl-piperidin-4-yloxy)-7(2-methoxy-ethoxy)-chinazolin,
(39) 4-[(3-Chlor-4-fluor-phenyl)amino]-6-(1-isopropyloxycarbonyl-piperidin-4-yloxy)-7-methoxy-chinazolin,
(40) 4-[(3-Chlor-4-fluor-phenyl)amino]-6-(cis-4-methylamino-cyclohexan-1-yloxy)-7-methoxy-chinazolin,
(41) 4-[(3-Chlor-4-fluor-phenyl)amino]-6-{cis-4-[N-(2-methoxy-acetyl)-N-methyl-amino]-cyclohexan-1-yloxy}-7-methoxy-chinazolin,
(42) 4-[(3-Ethinyl-phenyl)amino]-6-(piperidin-4-yloxy)-7-methoxy-chinazolin,
(43) 4-[(3-Ethinyl-phenyl)amino]-6-[1-(2-methoxy-acetyl)-piperidin-4-yloxy]-7-methoxy-chinazolin,
(44) 4-[(3-Ethinyl-phenyl)amino]-6-{1-[(morpholin-4-yl)carbonyl]-piperidin-4-yloxy}-7-methoxy-chinazolin,
(45) 4-[(3-Chlor-4-fluor-phenyl)amino]-6-{1-[(cis-2,6-dimethyl-morpholin-4-yl)carbonyl]-piperidin-4-yloxy}-7-methoxy-chinazolin,
(46) 4-[(3-Chlor-4-fluor-phenyl)amino]-6-{1-[(2-methyl-morpholin-4-yl)carbonyl]-piperidin-4-yloxy}-7-methoxy-chinazolin,
(47) 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin,
(48) 4-[(3-Chlor-4-fluor-phenyl)amino]-6-{1-[(N-methyl-N-2-methoxyethyl-amino)carbonyl]-piperidin-4-yloxy}-7-methoxy-chinazolin,
(49) 4-[(3-Chlor-4-fluor-phenyl)amino]-6-(1-ethyl-piperidin-4-yloxy)-7-methoxy-chinazolin,
(50) 4-[(3-Chlor-4-fluor-phenyl)amino]-6-{1-[(2-methoxyethyl)carbonyl]-piperidin-4-yloxy}-7-methoxy-chinazolin und
(51) 4-[(3-Chlor-4-fluor-phenyl)amino]-6-{1-[(3-methoxypropyl-amino)-carbonyl]-piperidin-4-yloxy}-7-methoxy-chinazolin,
deren Tautomere, deren Stereoisomere und deren Salze, insbesondere deren physiologisch verträgliche Salze mit anorganischen oder organischen Säuren oder Basen, zur Herstellung eines Arzneimittels zur Vorbeugung und Behandlung von entzündlichen Erkrankungen der Gelenke, wie rheumatoider Arthritis, von entzündlichen Erkrankungen, der Augen.

Bei dem erfindungsgemäßen Verfahren werden die genannten Verbindungen in Dosierungen von 0.001-10 mg/kg Körpergewicht, vorzugsweise bei 0.01-1,5 mg/kg eingesetzt, wobei die Gabe zweckmäßigerweise 1 bis 3 mal täglich erfolgt.

Die Wirkstoffe können oral, bukkal, parenteral, durch Inhalations-Zerstäubung, rektal oder topisch verabreicht werden. Eine parenterale Verabreichung kann subkutane, intravenöse, und intramuskuläre Injektionen und Infusionstechniken umfassen.

Zur Verabreichung können übliche Darreichungsformen verwendet werden, beispielsweise die in den vorstehend zu den Wirkstoffen zitierten genannten Darreichungsformen. Beispielsweise lassen sich die Wirkstoffe, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die Wirkstoffe können oral in einer breiten Vielfalt von verschiedenen Dosierungsformen verabreicht werden, beispielsweise können sie zusammen mit verschiedenen pharmazeutisch annehmbaren inerten Trägern in Form von Tabletten, Kapseln, Pastillen, Plätzchen, harten Bonbons, Pulvern, Zerstäubungen, wässrigen Suspensionen, Elixiren, Sirupen und dergleichen formuliert werden. Derartige Träger umfassen beispielsweise feste Verdünner oder Füllstoffe, sterile wässrige Medien und verschiedene nichttoxische organische Lösungsmittel. Zudem können derartige orale Formulierungen auf geeignete Weise mit Hilfe von verschiedenen, üblicherweise für diesen Zweck eingesetzten Agenzien gesüsst und/oder aromatisiert sein. Im allgemeinen sind die Wirkstoffe in solchen oralen Dosierungsformen mit Konzentrationsspiegeln vorhanden, deren Bereich, bezogen auf die Gesamtzusammensetzung, von etwa 0.5 Gew.-% bis etwa Gew.-90 % reicht, in Mengen, die ausreichen, um die gewünschten Dosierungseinheiten zu ergeben. Andere geeignete Dosierungsformen für die Wirkstoffe umfassen Formulierungen zur kontrollierten Freisetzung und Vorrichtungen, die den Fachpersonen auf dem betreffenden Gebiet wohlbekannt sind.

Zu Zwecken der parenteralen Verabreichung sind Lösungen der Wirkstoffe in Sesam- oder Erdnussöl oder in wässrigem Propylenglykol verwendbar, sowie sterile wässrige Lösungen der entsprechenden pharmazeutisch annehmbaren Salze. Derartige wässrige Lösungen sollten nötigenfalls auf geeignete Weise gepuffert und der flüssige Verdünner mit genügend Salz oder Glucose isotonisch gemacht werden. Besonders eignen sich diese bestimmten wässrigen Lösungen zum Zwecke von intravenösen, intramuskulären und subkutanen Injektionen. In diesem Zusammenhang sind die verwendeten sterilen wässrigen Medien nach gängigen, den Fachpersonen wohlbekannten Techniken leicht zu erhalten. Beispielsweise wird gewöhnlich destilliertes Wasser als flüssiger Verdünner verwendet, und das Endpräparat wird durch einen geeigneten Bakterienfilter wie ein Filter aus gesintertem Glas oder aus Kieselgur oder aus unglasiertem Porzellan geführt. Bevorzugte Filter dieses Typus umfassen die Berkefeld-, Chamberland- und Asbestscheiben-Metall-Seitzfilter, bei denen das Fluid mit Hilfe einer Saugpumpe in einen sterilen Behälter hinein gesaugt wird. Während der Herstellung dieser injizierbaren Lösungen sollten durchgehend die nötigen Verfahrensschritte vorgenommen werden, um zu sichern, dass die Endprodukte in sterilem Zustand erhalten werden. Zu Zwecken der transdermalen Verabreichung kann die Dosierungsform der bestimmten Verbindung oder Verbindungen beispielsweise Lösungen, Lotionen, Salben, Cremes, Gele, Zäpfchen, Formulierungen zur dauerhaften geschwindigkeitsbegrenzten Freisetzung und Vorrichtungen dazu umfassen. Derartige Dosierungsformen umfassen die bestimmte Verbindung bzw. die bestimmten Verbindungen und können Ethanol, Wasser, Eindringfördermittel und inerte Träger wie Gel-Erzeuger, Mineralöl, Emulgatoren, Benzylalkohol und dergleichen umfassen.
Eine inhalative Verabreichung erfolgt in Form von Pulverformulierungen mit Lactose und anderen Hilfsstoffen oder in Form wässriger Lösungen als Aerosol.
Die im Rahmen der erfindungsgemäßen Verwendung einsetzbaren Inhalationspulver können den Wirkstoff oder die Wirkstoffkombination entweder allein oder im Gemisch mit geeigneten physiologisch unbedenklichen Hilfsstoffen enthalten. Ist der Wirkstoff oder die Wirkstoffkombination im Gemisch mit physiologisch unbedenklichen Hilfsstoffen enthalten, können zur Darstellung dieser erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung.

Die im Rahmen der erfindungsgemäßen Verwendung einsetzbaren treibgashaltigen Inhaltionsaerosole können den Wirkstoff oder die Wirkstoffkombination im Treibgas gelöst oder in dispergierter Form enthalten. Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt fluorierten Derivaten des Methans, Ethans, Propans, Butans, Cyclopropans oder Cyclobutans. Die vorstehend genannten Treibgase können dabei allein oder in Mischungen derselben zur Verwendung kommen. Besonders bevorzugte Treibgase sind fluorierte Alkanderivate ausgewählt aus TG134a (1,1,1,2-Tetrafluorethan), TG227 (1,1,1,2,3,3,3-Heptafluorpropan) und Mischungen derselben.

Die im Rahmen der erfindungsgemäßen Verwendung einsetzbaren treibgashaltigen Inhalationsaerosole können ferner weitere Bestandteile wie Kosolventien, Stabilisatoren, oberflächenaktive Mittel (Surfactants), Antioxidantien, Schmiermittel sowie Mittel zur Einstellung des pH-Werts enthalten. All diese Bestandteile sind im Stand der Technik bekannt.

Erfolgt die inhalative Applikation des erfindungsgemäßen Wirkstoffs oder der Wirkstoffkombination in Form von treibgasfreien Lösungen oder Suspensionen kommen als Lösungsmittel wässrige oder alkoholische, bevorzugt ethanolische Lösungen in Betracht. Das Lösungsmittel kann ausschließlich Wasser sein oder es ist ein Gemisch aus Wasser und Ethanol. Der relative Anteil an Ethanol gegenüber Wasser ist nicht begrenzt, bevorzugt liegt die maximale Grenze jedoch bei bis zu 70 Volumenprozent, insbesondere bei bis zu 60 Volumenprozent und besonders bevorzugt bei bis zu 30 Volumenprozent. Die restlichen Volumenprozente werden von Wasser aufgefüllt. Die den Wirkstoff oder die Wirkstoffkombination enthaltenden Lösungen oder Suspensionen werden gegebenenfalls mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Wie bereits eingangs erwähnt, weisen die Verbindungen der allgemeinen Formel (I) und deren Salze wertvolle Eigenschaften auf, insbesondere eine antiinflammatorische Wirkung.

Beispielsweise wurden die Verbindungen
(A) 4-[(3-Chlor-4-fluor-phenyl)amino]-6-{1-[(morpholin-4-yl)carbonyl]-piperidin-4-yl-oxy}-7-methoxy-chinazolin,
(B) 4-[(3-Chlor-4-fluor-phenyl)amino]-6-{1-[(methoxymethyl)carbonyl]-piperidin-4-yl-oxy}-7-methoxy-chinazolin,
(C) 4-[(3-Chlor-4-fluor-phenyl)amino]-6-[1-(2-acetylamino-ethyl)-piperidin-4-yloxy]-7-methoxy-chinazolin,
(D) 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazölin,
(E) 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin,
(F) 4-[(3-Chlor-4-fluor-phenyl)amino]-6-(cis-4-acetylamino-cyclohexan-1-yloxy)-7-methoxy-chinazolin,
(G) 4-[(3-Ethinyl-phenyl)amino]-6-[1-(tert.-butyloxycarbonyl)-piperidin-4-yloxy]-7-methoxy-chinazolin,
(H) 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin,
(I) 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin,
(K) 4-[(3-Chlor-4-fluor-phenyl)amino]-6-{cis-4-[(morpholin-4-yl)carbonylamino]-cyclohexan-1-yloxy}-7-methoxy-chinazolin,
(L) 4-[(3-Chlor-4-fluor-phenyl)amino]-6-{1-[(morpholin-4-yl)carbonyl]-piperidin-4-yloxy}-7-(2-methoxy-ethoxy)-chinazolin und
(M) 4-[(3-Ethinyl-phenyl)amino]-6-(1-methyl-piperidin-4-yloxy)-7-methoxy-chinazolin
zur Untersuchung der antiinflammatorischen Wirkung dem folgenden Test unterworfen:

### Test 1: Hemmung der Rauch-induzierten Akkumulation von Granulozyten im Lungengewebe (Referenz)

### Lungenindikationen: Hemmung des Zigarettenrauch-induzierten Einstroms von neutrophilen Granulozyten in das Lungengewebe durch die o.g. EGF Rezeptor Kinase Hemmer

### Methode:

Männliche Ratten (Stamm: Sprague-Dawley) mit einem Gewicht von 250-300 g wurden 5 Tage lang dem Rauch von 8 Zigaretten pro Tag ausgesetzt. Die Tiere in der mit Verbindung A behandelten Gruppe erhielten täglich 30 min vor Beginn der Rauchexposition unter einer Narkose mit Isofluran eine intratracheale Gabe von 0.03 oder 0.1 mg/kg der Verbindung A in einem Volumen von 0.05 ml verabreicht. Am letzten Versuchstag wurden die Tiere 4 Stunden nach der letzten Rauchexposition getötet und das Lungengewebe entnommen. Aus jeder Lunge wurde eine Probe von 70 - 200 mg entnommen und in ein mit 1ml 0,5% Hexadecyltrimethylammoniumbromid vorbereitetes Reaktionsgefäß gegeben. Die Proben wurden 15 sec mit einem Ultraturrax homogenisiert. Die Homogenate wurden bei 15700 g in einer Eppendorf Tischzentrifuge 5 min bei Raumtemperatur abzentrifugiert. Von dem Überstand wurden 50 ml entnommen und mit 250 ml Phosphatpuffer (50mmol/l), der 0.197 mg/ml O-Dianisidin Dihydrochlorid enthielt, vermischt. Nach einer 10 minütigen Inkubation bei Raumtemperatur wurde mit einem Spektralphotometer bei einer Wellenlänge von 450 nm die Absorption gemessen.

Durch lineare Regression wurde die Dosis ermittelt, die zu einer Hemmung der MPO-Aktivität um 50% (= ID50) führte.

### Ergebnis:

Exposition von Zigarettenrauch führte bei Ratten zu einem Einstrom von neutrophilen Granulozyten in das Lungengewebe, gemessen durch den Gewebsgehalt an Myeloperoxidase, die spezifisch ist für neutrophile Granulozyten. Intratracheale Behandlung der Tiere mit dem EGFR Kinase Hemmer A bewirkte eine signifikante (p<0,005) Hemmung der Rauch-bedingten Akkumulation von Granulozyten und erzeugte damit eine antiinflammatorische Wirkung.

Weitere Ergebnisse sind in der folgenden Tabelle zusammengefaßt:

| **Wirkstoff** | **ID50** [mg/kg] |
|---|---|
| A | 0.100 |
| B | 0.010 |
| C | 0.030 |
| D | 0.020 |
| E | 0.400 |
| F | 0.015 |
| G | 0.010 |
| H | 0.150 |
| I | 0.150 |
| K | 0.130 |
| L | 0.400 |
| M | 0.070 |

## Patentansprüche

1. Verwendung von Chinazolinen ausgewählt aus der Gruppe bestehend aus
(1) 4-[(3-Chlor-4-fluor-phenyl)amino]-6-[1-(tert.-butyloxycarbonyl)-piperidin-4-yloxy]-7-methoxy-chinazolin,
(2) 4-[(3-Chlor-4-fluor-phenyl)amino]-6-(trans-4-amino-cyclohexan-1-yloxy)-7-methoxy-chinazolin,
(3) 4-[(3-Chlor-4-fluor-phenyl)amino]-6-(trans-4-methansulfonylamino-cyclohexan-1-yloxy)-7-methoxy-chinazolin,
(4) 4-[(3-Chlor-4-fluor-phenyl)amino]-6-(tetrahydropyran-3-yloxy)-7-methoxy-chinazolin,
(5) 4-[(3-Chlor-4-fluor-phenyl)amino]-6-(1-methyl-piperidin-4-yloxy)-7-methoxy-chinazolin,
(6) 4-[(3-Chlor-4-fluor-phenyl)amino]-6-{1-[(morpholin-4-yl)carbonyl]-piperidin-4-yl-oxy}-7-methoxy-chinazolin,
(7) 4-[(3-Chlor-4-fluor-phenyl)amino]-6-{1-[(methoxymethyl)carbonyl]-piperidin-4-yl-oxy}-7-methoxy-chinazolin,
(8) 4-[(3-Chlor-4-fluor-phenyl)amino]-6-(piperidin-3-yloxy)-7-methoxy-chinazolin,
(9) 4-[(3-Chlor-4-fluor-phenyl)amino]-6-[1-(2-acetylamino-ethyl)-piperidin-4-yloxy]-7-methoxy-chinazolin,
(10) 4-[(3-Chlor-4-fluor-phenyl)amino]-6-(tetrahydropyran-4-yloxy)-7-ethoxy-chinazolin,
(11) 4-[(3-Chlor-4-fluor-phenyl)amino]-6-((*S*)-tetrahydrofuran-3-yloxy)-7-hydroxy-chinazolin,
(12) 4-[(3-Chlor-4-fluor-phenyl)amino]-6-(tetrahydropyran-4-yloxy)-7-(2-methoxy-ethoxy)-chinazolin,
(13) 4-[(3-Chlor-4-fluor-phenyl)amino]-6-{trans-4-[(dimethylamino)sulfonylamino]-cyclohexan-1-yloxy}-7-methoxy-chinazolin,
(14) 4-[(3-Chlor-4-fluor-phenyl)amino]-6-{trans-4-[(morpholin-4-yl)carbonylamino]-cyclohexan-1-yloxy}-7-methoxy-chinazolin,
(15) 4-[(3-Chlor-4-fluor-phenyl)amino]-6-{trans-4-[(morpholin-4-yl)sulfonylamino]-cyclohexan-1-yloxy}-7-methoxy-chinazolin,
(16) 4-[(3-Chlor-4-fluor-phenyl)amino]-6-(tetrahydropyran-4-yloxy)-7-(2-acetylamino-ethoxy)-chinazolin,
(17) 4-[(3-Chlor-4-fluor-phenyl)amino]-6-(tetrahydropyran-4-yloxy)-7-(2-methansulfonylamino-ethoxy)-chinazolin,
(18) 4-[(3-Chlor-4-fluor-phenyl)amino]-6-{1-[(piperidin-1-yl)carbonyl]-piperidin-4-yloxy}-7-methoxy-chinazolin,
(19) 4-[(3-Chlor-4-fluor-phenyl)amino]-6-(1-aminocarbonylmethyl-piperidin-4-yloxy)-7-methoxy-chinazolin,
(20) 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin,
(21) 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin,
(22) 4-[(3-Chlor-4-fluor-phenyl)amino]-6-(cis-4-{N-[(morpholin-4-yl)sulfonyl]-N-methyl-amino}-cyclohexan-1-yloxy)-7-methoxy-chinazolin,
(23) 4-[(3-Chlor-4-fluor-phenyl)amino]-6-(trans-4-ethansulfonylamino-cyclohexan-1-yloxy)-7-methoxy-chinazolin,
(24) 4-[(3-Chlor-4-fluor-phenyl)amino]-6-(1-methansulfonyl-piperidin-4-yloxy)-7-ethoxy-chinazolin,
(25) 4-[(3-Chlor-4-fluor-phenyl)amino]-6-(1-methansulfonyl-piperidin-4-yloxy)-7-(2-methoxy-ethoxy)-chinazolin,
(26) 4-[(3-Chlor-4-fluor-phenyl)amino]-6-[1-(2-methoxy-acetyl)-piperidin-4-yloxy]-7-(2-methoxy-ethoxy)-chinazolin,
(27) 4-[(3-Chlor-4-fluor-phenyl)amino]-6-(cis-4-acetylamino-cyclohexan-1-yloxy)-7-methoxy-chinazolin,
(28) 4-[(3-Ethinyl-phenyl)amino]-6-[1-(tert.-butyloxycarbonyl)-piperidin-4-yloxy]-7-methoxy-chinazolin,
(29) 4-[(3-Ethinyl-phenyl)amino]-6-(tetrahydropyran-4-yloxy]-7-methoxy-chinazolin,
(30) 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin,
(31) 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin,
(32) 4-[(3-Chlor-4-fluor-phenyl)amino]-6-{cis-4-[(morpholin-4-yl)carbonylamino]-cyclohexan-1-yloxy}-7-methoxy-chinazolin,
(33) 4-[(3-Chlor-4-fluor-phenyl)amino]-6-{1-[2-(2-oxopyrrolidin-1-yl)ethyl]-piperidin-4-yloxy}-7-methoxy-chinazolin,
(34) 4-[(3-Chlor-4-fluor-phenyl)amino]-6-{1-[(morpholin-4-yl)carbonyl]-piperidin-4-yloxy}-7-(2-methoxy-ethoxy)-chinazolin,
(35) 4-[(3-Ethinyl-phenyl)amino]-6-(1-acetyl-piperidin-4-yloxy)-7-methoxy-chinazolin,
(36) 4-[(3-Ethinyl-phenyl)amino]-6-(1-methyl-piperidin-4-yloxy)-7-methoxy-chinazolin,
(37) 4-[(3-Ethinyl-phenyl)amino]-6-(1-methansulfonyl-piperidin-4-yloxy)-7-methoxy-chinazolin,
(38) 4-[(3-Chlor-4-fluor-phenyl)amino]-6-(1-methyl-piperidin-4-yloxy)-7(2-methoxy-ethoxy)-chinazolin,
(39) 4-[(3-Chlor-4-fluor-phenyl)amino]-6-(1-isopropyloxycarbonyl-piperidin-4-yloxy)-7-methoxy-chinazolin,
(40) 4-[(3-Chlor-4-fluor-phenyl)amino]-6-(cis-4-methylamino-cyclohexan-1-yloxy)-7-methoxy-chinazolin,
(41) 4-[(3-Chlor-4-fluor-phenyl)amino]-6-{cis-4-[N-(2-methoxy-acetyl)-N-methyl-amino]-cyclohexan-1-yloxy}-7-methoxy-chinazolin,
(42) 4-[(3-Ethinyl-phenyl)amino]-6-(piperidin-4-yloxy)-7-methoxy-chinazolin,
(43) 4-[(3-Ethinyl-phenyl)amino]-6-[1-(2-methoxy-acetyl)-piperidin-4-yloxy]-7-methoxy-chinazolin,
(44) 4-[(3-Ethinyl-phenyl)amino]-6-{1-[(morpholin-4-yl)carbonyl]-piperidin-4-yloxy}-7-methoxy-chinazolin,
(45) 4-[(3-Chlor-4-fluor-phenyl)amino]-6-{1-[(cis-2,6-dimethyl-morpholin-4-yl)carbonyl]-piperidin-4-yloxy}-7-methoxy-chinazolin,
(46) 4-[(3-Chlor-4-fluor-phenyl)amino]-6-{1-[(2-methyl-morpholin-4-yl)carbonyl]-piperidin-4-yloxy}-7-methoxy-chinazolin,
(47) 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin,
(48) 4-[(3-Chlor-4-fluor-phenyl)amino]-6-{1-[(N-methyl-N-2-methoxyethyl-amino)carbonyl]-piperidin-4-yloxy}-7-methoxy-chinazolin,
(49) 4-[(3-Chlor-4-fluor-phenyl)amino]-6-(1-ethyl-piperidin-4-yloxy)-7-methoxy-chinazolin,
(50) 4-[(3-Chlor-4-fluor-phenyl)amino]-6-{1-[(2-methoxyethyl)carbonyl]-piperidin-4-yloxy}-7-methoxy-chinazolin und
(51) 4-[(3-Chlor-4-fluor-phenyl)amino]-6-{1-[(3-methoxypropyl-amino)-carbonyl]-piperidin-4-yloxy}-7-methoxy-chinazolin,
deren Tautomere, deren Stereoisomere und deren Salze, insbesondere deren physiologisch verträgliche Salze mit anorganischen oder organischen Säuren oder Basen, zur Herstellung eines Arzneimittels zur Vorbeugung und Behandlung entzündlicher Erkrankungen der Gelenke und der Augen.

## Claims

1. Use of quinazolines selected from the group comprising
(1) 4-[(3-chloro-4-fluoro-phenyl)amino]-6-[1-(tert.-butyloxycarbonyl)-piperidin-4-yloxy]-7-methoxy-quinazoline,
(2) 4-[(3-chloro-4-fluoro-phenyl)amino]-6-(trans-4-amino-cyclohexan-1-yloxy)-7-methoxy-quinazoline,
(3) 4-[(3-chloro-4-fluoro-phenyl)amino]-6-(trans-4-methanesulphonylamino-cyclohexan-1-yloxy)-7-methoxy-quinazoline,
(4) 4-[(3-chloro-4-fluoro-phenyl)amino]-6-(tetrahydropyran-3-yloxy)-7-methoxyquinazoline,
(5) 4-[(3-chloro-4-fluoro-phenyl)amino]-6-(1-methyl-piperidin-4-yloxy)-7-methoxy-quinazoline,
(6) 4-[(3-chloro-4-fluoro-phenyl)amino]-6-{1-[(morpholin-4-yl)carbonyl]-piperidin-4-yloxy}-7-methoxy-quinazoline,
(7) 4-[(3-chloro-4-fluoro-phenyl)amino]-6-{1-[(methoxymethyl)carbonyl]-piperidin-4-yloxy}-7-methoxy-quinazoline,
(8) 4-[(3-chloro-4-fluoro-phenyl)amino]-6-(piperidin-3-yloxy)-7-methoxyquinazoline,
(9) 4-[(3-chloro-4-fluoro-phenyl)amino]-6-[1-(2-acetylamino-ethyl)-piperidin-4-yloxy]-7-methoxy-quinazoline,
(10) 4-[(3-chloro-4-fluoro-phenyl)amino]-6-(tetrahydropyran-4-yloxy)-7-ethoxy-quinazoline,
(11) 4-[(3-chloro-4-fluoro-phenyl)amino]-6-((*S*)-tetrahydrofuran-3-yloxy)-7-hydroxy-quinazoline,
(12) 4-[(3-chloro-4-fluoro-phenyl)amino]-6-(tetrahydropyran-4-yloxy)-7-(2-methoxy-ethoxy)-quinazoline,
(13) 4-[(3-chloro-4-fluoro-phenyl)amino]-6-{trans-4-[(dimethylamino)sulphonylamino]-cyclohexan-1-yloxy}-7-methoxyquinazoline,
(14) 4-[(3-chloro-4-fluoro-phenyl)amino]-6-{trans-4-[(morpholin-4-yl)carbonylamino]-cyclohexan-1-yloxy}-7-methoxy-quinazoline,
(15) 4-[(3-chloro-4-fluoro-phenyl)amino]-6-{trans-4-[(morpholin-4-yl)sulphonylamino]-cyclohexan-1-yloxy}-7-methoxy-quinazoline,
(16) 4-[(3-chloro-4-fluoro-phenyl)amino]-6-(tetrahydropyran-4-yloxy)-7-(2-acetylamino-ethoxy)-quinazoline,
(17) 4-[(3-chloro-4-fluoro-phenyl)amino]-6-(tetrahydropyran-4-yloxy)-7-(2-methanesulphonylamino-ethoxy)-quinazoline,
(18) 4-[(3-chloro-4-fluoro-phenyl)amino]-6-{1-[(piperidin-1-yl)carbonyl]-piperidin-4-yloxy}-7-methoxy-quinazoline,
(19) 4-[(3-chloro-4-fluoro-phenyl)amino]-6-(1-aminocarbonylmethyl-piperidin-4-yloxy)-7-methoxy-quinazoline,
(20) 4-[(3-chloro-4-fluoro-phenyl)amino]-6-(cis-4-{N-[(tetrahydropyran-4-yl)carbonyl]-N-methyl-amino}-cyclohexan-1-yloxy)-7-methoxy-quinazoline,
(21) 4-[(3-chloro-4-fluoro-phenyl)amino]-6-(cis-4-{N-[(morpholin-4-yl)carbonyl]-N-methyl-amino}-cyclohexan-1-yloxy)-7-methoxy-quinazoline,
(22) 4-[(3-chloro-4-fluoro-phenyl)amino]-6-(cis-4-{N-[(morpholin-4-yl)sulphonyl]-N-methyl-amino}-cyclohexan-1-yloxy)-7-methoxy-quinazoline,
(23) 4-[(3-chloro-4-fluoro-phenyl)amino]-6-(trans-4-ethanesulphonylamino-cyclohexan-1-yloxy)-7-methoxy-quinazoline,
(24) 4-[(3-chloro-4-fluoro-phenyl)amino]-6-(1-methanesulphonyl-piperidin-4-yloxy)-7-ethoxy-quinazoline,
(25) 4-[(3-chloro-4-fluoro-phenyl)amino]-6-(1-methanesulphonyl-piperidin-4-yloxy)-7-(2-methoxy-ethoxy)-quinazoline,
(26) 4-[(3-chloro-4-fluoro-phenyl)amino]-6-[1-(2-methoxy-acetyl)-piperidin-4-yloxy]-7-(2-methoxy-ethoxy)-quinazoline,
(27) 4-[(3-chloro-4-fluoro-phenyl)amino]-6-(cis-4-acetylamino-cyclohexan-1-yloxy)-7-methoxy-quinazoline,
(28) 4-[(3-ethynyl-phenyl)amino]-6-[1-(tert.-butyloxycarbonyl)-piperidin-4-yloxy]-7-methoxy-quinazoline,
(29) 4-[(3-ethynyl-phenyl)amino]-6-(tetrahydropyran-4-yloxy]-7-methoxyquinazoline,
(30) 4-[(3-chloro-4-fluoro-phenyl)amino]-6-(cis-4-{N-[(piperidin-1-yl)carbonyl]-N-methyl-amino}-cyclohexan-1-yloxy)-7-methoxy-quinazoline,
(31) 4-[(3-chloro-4-fluoro-phenyl)amino]-6-(cis-4-{N-[(4-methyl-piperazin-1-yl)carbonyl]-N-methyl-amino}-cyclohexan-1-yloxy)-7-methoxy-quinazoline,
(32) 4-[(3-chloro-4-fluoro-phenyl)amino]-6-{cis-4-[(morpholin-4-yl)carbonylamino]-cyclohexan-1-yloxy}-7-methoxy-quinazoline,
(33) 4-[(3-chloro-4-fluoro-phenyl)amino]-6-{1-[2-(2-oxopyrrolidin-1-yl)ethyl]-piperidin-4-yloxy}-7-methoxy-quinazoline,
(34) 4-[(3-chloro-4-fluoro-phenyl)amino]-6-{1-[(morpholin-4-yl)carbonyl]-piperidin-4-yloxy}-7-(2-methoxy-ethoxy)-quinazoline,
(35) 4-[(3-ethynyl-phenyl)amino]-6-(1-acetyl-piperidin-4-yloxy)-7-methoxyquinazoline,
(36) 4-[(3-ethynyl-phenyl)amino]-6-(1-methyl-piperidin-4-yloxy)-7-methoxyquinazoline,
(37) 4-[(3-ethynyl-phenyl)amino]-6-(1-methanesulphonyl-piperidin-4-yloxy)-7-methoxy-quinazoline,
(38) 4-[(3-chloro-4-fluoro-phenyl)amino]-6-(1-methyl-piperidin-4-yloxy)-7-(2-methoxy-ethoxy)-quinazoline,
(39) 4-[(3-chloro-4-fluoro-phenyl)amino]-6-(1-isopropyloxycarbonyl-piperidin-4-yloxy)-7-methoxy-quinazoline,
(40) 4-[(3-chloro-4-fluoro-phenyl)amino]-6-(cis-4-methylamino-cyclohexan-1-yloxy)-7-methoxy-quinazoline,
(41) 4-[(3-chloro-4-fluoro-phenyl)amino]-6-{cis-4-[N-(2-methoxy-acetyl)-N-methyl-amino]-cyclohexan-1-yloxy}-7-methoxy-quinazoline,
(42) 4-[(3-ethynyl-phenyl)amino]-6-(piperidin-4-yloxy)-7-methoxy-quinazoline,
(43) 4-[(3-ethynyl-phenyl)amino]-6-[1-(2-methoxy-acetyl)-piperidin-4-yloxy]-7-methoxy-quinazoline,
(44) 4-[(3-ethynyl-phenyl)amino]-6-{1-[(morpholin-4-yl)carbonyl]-piperidin-4-yloxy}-7-methoxy-quinazoline,
(45) 4-[(3-chloro-4-fluoro-phenyl)amino]-6-{1-[(cis-2,6-dimethyl-morpholin-4-yl)carbonyl]-piperidin-4-yloxy}-7-methoxy-quinazoline,
(46) 4-[(3-chloro-4-fluoro-phenyl)amino]-6-{1-[(2-methyl-morpholin-4-yl)carbonyl]-piperidin-4-yloxy}-7-methoxy-quinazoline,
(47) 4-[(3-chloro-4-fluoro-phenyl)amino]-6-{1-[(S,S)-(2-oxa-5-aza-bicyclo[2.2.1]hept-5-yl)carbonyl]-piperidin-4-yloxy}-7-methoxy-quinazoline,
(48) 4-[(3-chloro-4-fluoro-phenyl)amino]-6-{1-[(N-methyl-N-2-methoxyethyl-amino)carbonyl]-piperidin-4-yloxy}-7-methoxy-quinazoline,
(49) 4-[(3-chloro-4-fluoro-phenyl)amino]-6-(1-ethyl-piperidin-4-yloxy)-7-methoxyquinazoline,
(50) 4-[(3-chloro-4-fluoro-phenyl)amino]-6-{1-[(2-methoxyethyl)carbonyl]-piperidin-4-yloxy}-7-methoxy-quinazoline and
(51) 4-[(3-chloro-4-fluoro-phenyl)amino]-6-{1-[(3-methoxypropyl-amino)-carbonyl]-piperidin-4-yloxy}-7-methoxy-quinazoline,
the tautomers, stereoisomers and salts thereof, particularly the physiologically acceptable salts thereof with inorganic or organic acids or bases, for preparing a pharmaceutical composition for the prevention and treatment of inflammatory diseases of the joints and eyes.

## Revendications

1. Utilisation de quinazolines sélectionnées parmi le groupe constitué de
(1) 4-[(3-chloro-4-fluoro-phényl)amino]-6-[1-(tert.-butyloxycarbonyl)-pipéridin-4-yloxy]-7-méthoxy-quinazoline,
(2) 4-[(3-chloro-4-fluoro-phényl)amino]-6-(trans-4-amino-cyclohexane-1-yloxy)-7-méthoxy-quinazoline,
(3) 4-[(3-chloro-4-fluoro-phényl)amino]-6-(trans-4-méthansulfonylamino-cyclohexane-1-yloxy)-7-méthoxy-quinazoline,
(4) 4-[(3-chloro-4-fluoro-phényl)amino]-6-(tétrahydropyran-3-yloxy)-7-méthoxy-quinazoline,
(5) 4-[(3-chloro-4-fluoro-phényl)amino]-6-(1-méthyl-pipéridin-4-yloxy)-7-méthoxy-quinazoline,
(6) 4-[(3-chloro-4-fluoro-phényl)amino]-6-{1-[(morpholin-4-yl)carbonyl]-pipéridin-4-yloxy}-7-méthoxy-quinazoline,
(7) 4-[(3-chloro-4-fluoro-phényl)amino]-6-{1-[(méthoxyméthyl)carbonyl]-pipéridin-4-yloxy}-7-méthoxy-quinazoline,
(8) 4-[(3-chloro-4-fluoro-phényl)amino]-6-(pipéridin-3-yloxy)-7-méthoxy-quinazoline,
(9) 4-[(3-chloro-4-fluoro-phényl)amino]-6-[1-(2-acétylamino-éthyl)-pipéridin-4-yloxy]-7-méthoxy-quinazoline,
(10) 4-[(3-chloro-4-fluoro-phényl)amino]-6-(tétrahydropyran-4-yloxy)-7-éthoxy-quinazoline,
(11) 4-[(3-chloro-4-fluoro-phényl)amino]-6-((*S*)-tétrahydrofuran-3-yloxy)-7-hydroxy-quinazoline,
(12) 4-[(3-chloro-4-fluoro-phényl)amino]-6-(tétrahydropyran-4-yloxy)-7-(2-méthoxy-éthoxy)-quinazoline,
(13) 4-[(3-chloro-4-fluoro-phényl)amino]-6-{trans-4-[(diméthylamino)sulfonylamino]-cyclohexan-1-yloxy}-7-méthoxy-quinazoline,
(14) 4-[(3-chloro-4-fluoro-phényl)amino]-6-{trans-4-[(morpholin-4-yl)carbonylamino]-cyclohexan-1-yloxy}-7-méthoxy-quinazoline,
(15) 4-[(3-chloro-4-fluoro-phényl)amino]-6-{trans-4-[(morpholin-4-yl)sulfonylamino]-cyclohexan-1-yloxy}-7-méthoxy-quinazoline,
(16) 4-[(3-chloro-4-fluoro-phényl)amino]-6-(tétrahydropyran-4-yloxy)-7-(2-acétylamino-éthoxy)-quinazoline,
(17) 4-[(3-chloro-4-fluoro-phényl)amino]-6-(tétrahydropyran-4-yloxy)-7-(2-méthanesulfonylamino-éthoxy)-quinazoline,
(18) 4-[(3-chloro-4-fluoro-phényl)amino]-6-{1-[(pipéridine-1-yl)carbonyl]-pipéridin-4-yloxy}-7-méthoxy-quinazoline,
(19) 4-[(3-chloro-4-fluoro-phényl)amino]-6-(1-aminocarbonylméthyl-pipéridin-4-yloxy)-7-méthoxy-quinazoline,
(20) 4-[(3-chloro-4-fluoro-phényl)amino]-6-(cis-4-{N-[(tétrahydropyran-4-yl)carbonyl]-N-méthyl-amino}-cyclohexan-1-yloxy)-7-méthoxy-quinazoline,
(21) 4-[(3-chloro-4-fluoro-phényl)amino]-6-(cis-4-{N-[(morpholin-4-yl)carbonyl]-N-méthyl-amino}-cyclohexan-1-yloxy)-7-méthoxy-quinazoline,
(22) 4-[(3-chloro-4-fluoro-phényl)amino]-6-(cis-4-{N-[(morpholin-4-yl)sulfonyl]-N-méthyl-amino}-cyclohexan-1-yloxy)-7-méthoxy-quinazoline,
(23) 4-[(3-chloro-4-fluoro-phényl)amino]-6-(trans-4-éthanesulfonylamino-cyclohexan-1-yloxy)-7-méthoxy-quinazoline,
(24) 4-[(3-chloro-4-fluoro-phényl)amino]-6-(1-méthanesulfonyl-pipéridin-4-yloxy)-7-éthoxy-quinazoline,
(25) 4-[(3-chloro-4-fluoro-phényl)amino]-6-(1-méthanesulfonyl-pipéridin-4-yloxy)-7-(2-méthoxy-éthoxy)-quinazoline,
(26) 4-[(3-chloro-4-fluoro-phényl)amino]-6-[1-(2-méthoxy-acétyl)-pipéridin-4-yloxy)-7-(2-méthoxy-éthoxy)-quinazoline,
(27) 4-[(3-chloro-4-fluoro-phényl)amino]-6-(cis-4-acétylamino-cyclohexan-1-yloxy)-7-méthoxy-quinazoline,
(28) 4-[(3-éthinyl-phényl)amino]-6-[1-(tert.-butyloxycarbonyl)-pipéridin-4-yloxy]-7-méthoxy-quinazoline,
(29) 4-[(3-éthinyl-phényl)amino]-6-(tétrahydropyran-4-yloxy)-7-méthoxy-quinazoline,
(30) 4-[(3-chloro-4-fluoro-phényl)amino]-6-(cis-4-{N-[(pipéridin-1-yl)carbonyl]-N-méthyl-amino}-cyclohexan-1-yloxy)-7-méthoxy-quinazoline,
(31) 4-[(3-chloro-4-fluoro-phényl)amino]-6-(cis-4-{N-[(4-méthyl-pipérazin-1-yl)carbonyl]-N-méthyl-amino}-cyclohexan-1-yloxy)-7-méthoxy-quinazoline,
(32) 4-[(3-chloro-4-fluoro-phényl)amino]-6-{cis-4-[(morpholin-4-yl)carbonylamino]-cyclohexan-1-yloxy}-7-méthoxy-quinazoline,
(33) 4-[(3-chloro-4-fluoro-phényl)amino]-6-{1-[2-(2-oxopyrrolidin-1-yl)éthyl]-pipéridin-4-yloxy}-7-méthoxy-quinazoline,
(34) 4-[(3-chloro-4-fluoro-phényl)amino]-6-{1-[(morpholin-4-yl)carbonyl]-pipéridin-4-yloxy}-7-(2-méthoxy-éthoxy)-quinazoline,
(35) 4-[(3-éthinyl-phényl)amino]-6-(1-acétyl-pipéridin-4-yloxy)-7-méthoxy-quinazoline,
(36) 4-[(3-éthinyl-phényl)amino]-6-(1-méthyl-pipéridin-4-yloxy)-7-méthoxy-quinazoline,
(37) 4-[(3-éthinyl-phényl)amino]-6-(1-méthanesulfonyl-pipéridin-4-yloxy)-7(2-méthoxy-éthoxy)-quinazoline,
(38) 4-[(3-chloro-4-fluoro-phényl)amino]-6-(1-méthyl-pipéridin-4-yloxy)-7(2-méthoxy-éthoxy)-quinazoline,
(39) 4-[(3-chloro-4-fluoro-phényl)amino]-6-(1-isopropyloxycarbonyl-pipéridin-4-yloxy)-7-méthoxy-quinazoline,
(40) 4-[(3-chloro-4-fluoro-phényl)amino]-6-(cis-4-méthylamino-cyclohexan-1-yloxy)-7-méthoxy-quinazoline,
(41) 4-[(3-chloro-4-fluoro-phényl)amino]-6-{cis-4-[N-(2-méthoxy-acétyl)-N-méthyl-amino]-cyclohexan-1-yloxy}-7-méthoxy-quinazoline,
(42) 4-[(3-éthinyl-phényl)amino]-6-(pipéridin-4-yloxy)-7-méthoxy-quinazoline,
(43) 4-[(3-éthinyl-phényl)amino]-6-[1-(2-méthoxy-acétyl)-pipéridin-4-yloxy)-7-méthoxy-quinazoline,
(44) 4-[(3-éthinyl-phényl)amino]-6-{1-[(morpholin-4-yl)carbonyl]-pipéridin-4-yloxy}-7-méthoxy-quinazoline,
(45) 4-[(3-chloro-4-fluoro-phényl)amino]-6-{1-[(cis-2,6-diméthyl-morpholine-4-yl)carbonyl]-pipéridin-4-yloxy}-7-méthoxy-quinazoline,
(46) 4-[(3-chloro-4-fluoro-phényl)amino]-6-{1-[(2-méthyl-morpholin-4-yl)carbonyl]-pipéridin-4-yloxy}-7-méthoxy-quinazoline,
(47) 4-[(3-chloro-4-fluoro-phényl)amino]-6-{1-[(S,S)-(2-oxa-5-aza-bicyclo[2.2.1]hept-5-yl)carbonyl]-pipéridin-4-yloxy}-7-méthoxy-quinazoline,
(48) 4-[(3-chloro-4-fluoro-phényl)amino]-6-{1-[(N-méthyl-N-2-méthoxyéthyl-amino)carbonyl]-pipéridin-4-yloxy}-7-méthoxy-quinazoline,
(49) 4-[(3-chloro-4-fluoro-phényl)amino]-6-(1-éthyl-pipéridin-4-yloxy)-7-méthoxy-quinazoline,
(50) 4-[(3-chloro-4-fluoro-phényl)amino]-6-{1-[(2-méthoxyéthyl)carbonyl]-pipéridin-4-yloxy}-7-méthoxy-quinazoline et
(51) 4-[(3-chloro-4-fluoro-phényl)amino]-6-{1-[(3-méthoxypropyl-amino)carbonyl]-pipéridin-4-yloxy}-7-méthoxy-quinazoline,
leurs tautomères, stéréoisomères et sels, en particulier leurs sels physiologiquement acceptables avec des acides ou bases inorganiques ou organiques, pour fabriquer un médicament pour la prévention et le traitement de maladies inflammatoires des articulations et des yeux.
